# EUROPÄISCHE PATENTANMELDUNG

(11) **EP 1 500 388 A1**
(43) Veröffentlichungstag der Anmeldung: **26.01.2005**
(21) Anmeldenummer: 04010735.1
(22) Anmeldetag: 06.05.2004
(51) Int. Cl.: A61K 7/32, A61K 7/38

(54) **Klares O/W-Microemulsionsgel als Antitranspiranz**

(30) Priorität: 19.07.2003 DE 10332928
(71) Anmelder: Beiersdorf AG, 20253 Hamburg (DE)
(72) Erfinder: Kux, Ulrich, Dr., 22559 Hamburg (DE); Diec, Khiet Hien, 20251 Hamburg (DE)

(57) **Zusammenfassung**

Die vorliegende Erfindung betrifft eine optisch klar transluzente bis transparente Antitranspirantformulierung, aus einem Öl-in-Wasser-Emulsionsgel, insbesondere aus einem Öl-in-Wasser-Mikroemulsionsgel, und hat eine Viskosität von mindesten 5000 mPa s mit einem hohen Gehalt an Antitranspirantsalz.

## Beschreibung

Die vorliegende Erfindung betrifft eine optisch klar transluzente bis transparente Antitranspirantformulierung, aus einem ÖI-in-Wasser-Emulsionsgel, insbesondere aus einem ÖI-in-Wasser-Mikroemulsionsgel, und hat eine Viskosität von mindesten 5000 mPa s mit einem hohen Gehalt an Antitranspirantsalz.

Die menschliche Haut ist mit zwei bis d rei M illionen S chweißdrüsen d urchsetzt. D iese sind Tag und Nacht im Einsatz, um Feuchtigkeit an die Hautoberfläche zu befördern und so der Überhitzung des Organismus vorzubeugen. Der austretende Schweiß sorgt bei seiner Verdunstung für die notwendige Kühlung.
Täglich werden im Normalfall 0,5 bis 1 Liter Schweiß produziert. Bei Belastung des Körpers und erhöhtem Stoffwechsel kann es ein Vielfaches an produziertem Schweiß sein. Im Laufe der Entwicklung eines Menschen kommt es zur Ausbildung zweier Arten von Schweißdrüsen. Von Geburt an besitzt der Mensch nur ekkrine Schweißdrüsen (kleine Schweißdrüsen), mit Beginn der Pubertät jedoch kommt es hauptsächlich im Bereich der Achseln und im Anal- und Genitalbereich zur Ausbildung der apokrinen Schweißdrüsen (große Schweißdrüsen). Erst diese sorgen in Zusammenhang mit Hautbakterien, die den geruchlosen Schweiß zersetzen, zu den bekannten unangenehmen Gerüchen. Der Schweißgeruch ist personenspezifisch und bei jedem Menschen unterschiedlich ausgeprägt. Durch einfaches Waschen lässt sich bei den meisten Menschen nur eine kurzfristige Besserung erzielen, so dass es oft genug nicht ohne die Anwendung von Deowirkstoffen geht.
Um eine Deowirkung zu erreichen gibt es verschiedene Wege, die im Normalfall kombiniert angewendet werden.

Der Einsatz von Antitranspirantien, die die Schweißproduktion durch Blockierung der Schweißdrüsenausgänge verhindern, ist schon lange bekannt. Verwendung finden hier in aller Regel Aluminium- und Aluminium/Zirkoniumsalze. Die verringerte Schweißproduktion hat nach neuesten Erkenntnissen keine Auswirkung auf den Organismus, da die 'Kühlwirkung' zum großen Teil über das 'Schwitzen' der anderen Hautpartien (ekkrine Drüsen) erfolgt.
Die Hemmung des Bakterienwachstums durch Bakteriostatika im Bereich der mit apokrinen Schweißdrüsen durchsetzten Hautzonen ist nicht unbedenklich und führt zum Teil zu starken Reizungen und allergischen Reaktionen.
Als Bakteriozid wirkt auch der Alkohol (Ethanol), der in vielen der herkömmlichen Deoprodukte enthalten ist. Auch hier sind Nebenwirkungen häufig.
Zur Maskierung des Schweißgeruchs sind in der Regel Duftstoffe bzw. Parfümstoffe in den Deozubereitungen vorhanden. Diese wirken zum Teil auch bakteriostatisch, haben aber bei vielen Anwendern ähnliche Nebenwirkungen wie die Bakteriostatika.
Aufgrund der zuvor beschriebenen Wirkungsweise und den damit einhergehenden Nebenwirkungen sind die Bestrebungen groß, Deoprodukte zu entwickeln, die keine Nebenwirkungen aufweisen. Der Trend geht also eindeutig in Richtung Kombinationsprodukt, bei dem eine desodorierende und antitranspirante Wirkung mit hautpflegender Wirkung einhergeht.
Solche Pflegedeos auf Basis von O/W-Emulsionen sind schon im Markt eingeführt, die Handhabung, sprich die Aufbringung auf die Haut, lässt aber noch stark zu wünschen übrig.

Vor allem aus ästhetischen Gründen werden transparente und transluzente Produkte von vielen Verbrauchern bevorzugt. Die Kombination dieses Merkmals mit dem Wunsch nach gut wirksamen Antitranspirantprodukten ließ sich bislang nur mit wässrig-alkoholischen Rezepturen realisieren. Diese Formulierungen bestehen praktisch nur aus Wasser und Alkohol als Medium, Deo- und Antitranspirantmittel als Wirkstoffe sowie Parfüm, Löslichkeitsvermittler und Verdicker (zumeist auf Kohlenhydratbasis) als zusätzliche Agenzien. Sie werden vom Verbraucher als frisch und kühlend empfunden, sind aber gleichzeitig mit einer ganzen Reihe an Mankos behaftet. So ist die Applikation vor allem auf frisch rasierter Haut durch den Alkoholgehalt mit Unverträglichkeiten verbunden. Ein weiterer großer Nachteil ist die Tatsache, dass in derartige Systeme keine größeren Ölmengen eingearbeitet werden können. Durch den für eine hoch-effektive Wirkleistung erforderlichen hohen Gehalt an Antitranspirantsalz verbleibt nach der Applikation auf der Haut ein weißer Rückstand, der vom Verbraucher als überaus störend empfunden wird. Durch die technologisch bedingte Abwesenheit einer ausreichendend großen Ölphase kann dieser allerdings nicht kaschiert werden. Darüber hinaus führt die Verwendung von Kohlenhydrat-Verdickern zu einer gewissen Klebrigkeit des Produktes nach dem Verdunsten des Alkohols. Zusammenfassend kann man sagen, dass wässrigalkoholische Rezepturen nicht als Grundlage für die Einarbeitung hoher Gehalte an Antitranspirantwirkern (Aluminium- bzw. Aluminium/Zirkonium-Komplexe) geeignet sind.

Die Lösung all dieser Nachteile ließ lange auf sich warten. Erst in jüngster Zeit sind auch kosmetisch ansprechende alkoholfrei-transparente Produkte möglich, die auf sog. Mikroemulsionen basieren. Diese haben den großen Vorteil, dass man auch größere Mengen an verschiedenen Ölen - mit all den oben beschriebenen positiven Effekten für den Verbraucher - stabil einarbeiten kann. Formulierungen dieser Art sind prinzipiell mittels Phaseninversionstemperatur-Technologie (PIT) oder Hochdruckhomogenisierung zugänglich. Die notwendige Stabilität des Emulgatorsystems gegenüber hohen Konzentrationen an Antitranspirantsalzen stellt jedoch hohe Anforderungen an die Formulierungskunst des Produktentwicklers. Um eine Transparenz zu erreichen müssen die Brechungsindizes der Ölphase und der Wasserphase nahezu identisch sein. Schon eine Abweichung über 0,0004 führt zur Trübung der Formulierung. Die Herstellung von transparenten Formulierungen wird in WO92/05767 beschrieben.
Neben den Wasser-in-Silicon-Emulsionsgelen sind auch wasserfreie Oleogele und auf Carbomeren basierende Hydrogele marktüblich.

Viele Verbraucher favorisieren für die Auftragung von Antitranspirantien die Produktform der sogenannte Roll-on oder Deo-Stifte, da sich mit ihnen das Füllgut fein verteilt in die Achselregion aufbringen lässt, ohne dass die Finger mit diesem in Berührung gebracht werden müssen. Gegenüber Aerosolen besteht weiterhin der ökologische Vorteil, dass Roll-on und Deo-Stick ohne die Verwendung von Treibmitteln (verflüssigte Gase) auskommen.

Aufgabe der vorliegenden Erfindung ist es daher, den Stand der Technik zu bereichern und seinen Nachteilen abzuhelfen.

### Die Formulierung

Erstaunlicherweise lassen sich als Formulierung für die oben genannten Aufgaben optisch klar transluzente bis transparente, alkoholfreie Emulsionsgele, beruhend auf Emulsionen vom Typ ÖI-in-Wasser, insbesondere Mikroemulsionsgele vom Typ ÖI-in-Wasser, umfassend eine Ölphase und eine Wasserphase, enthaltend:
- einen oder mehrere O/W-Emulgatoren und/oder ein oder mehrere Vernetzer, insbesondere Polyole,
- gewünschtenfalls ferner enthaltend einen oder mehrere W/O-Emulgatoren,
- einen Emulgatorgehalt kleiner als 20 Gew.-%, bezogen auf das Gesamtgewicht der Emulsion,
- einen Antitranspirantgehalt von 5 bis 40 Gew.-%, bezogen auf das Gesamtgewicht der Emulsion, aufweisend,
- ein Polyolgehalt von mindestens 1 bis 30 Gew.-%, bezogen auf das Gesamtgewicht der Emulsion,
erhältlich auf die Weise, dass man ein Gemisch aus den Grundkomponenten, umfassend Wasserphase, Ölphase, erfindungsgemäßen O/W-Emulgatoren, gewünschtenfalls W/O-Emulgatoren, sowie gewünschtenfalls weiteren Hilfs-, Zusatz- und/oder Wirkstoffen auf eine Temperatur bringt, bei der alle Komponenten aufgelöst sind und hernach auf Raumtemperatur unter Rühren abkühlt, ohne die Emulsion zu homogenisieren
und bei welcher die Tröpfchen der diskontinuierlichen Ölphase durch Polyole miteinander verbunden sind, verwenden.

Erfindungsgemäße transparente Gele haben eine niedrige Viskosität, eignen sich vorzüglich als Vehikel für verschiedenste Wirkstoffe, insbesondere lipidlösliche Wirkstoffe und zeichnen sich darüber hinaus durch vorzügliche Haut- und Schleimhautverträglichkeit aus.
Die in der Erfindung benötigten Vernetzersubstanzen sind die Polyole, insbesondere die Triole, welche ein eingenständiges Gelnetzwerk bilden und ein Zusammenhalt des Netzes mit den Emulsionströpfchen in den Knotenpunkten des Netzes bewirken.

Alle Bestandteile - bis auf Wasser und Duftstoffe - der erfindungsgemäßen Emulsionsgele sind schwerflüchtig, d.h. sie weisen im reinen Zustand einen geringen Dampfdruck bei 25 °C auf, wodurch ein Eintrocknen sowie Kristallbildung unterbunden wird.

Vorteilhaft im Sinne der Erfindung ist es, wenn die ÖI-in-Wasser-Emulsion optisch klar transluzent bis transparent escheint und eine Tröpfchengröße kleiner 100 nm aufweist. Emulsionen mit mittleren Tröpfchengrößen unterhalb von 100 nm werden nach Stand der Technik als Mikroemulsionen bezeichnet.

Vorteilhaft wird oder werden der oder die O/W-Emulgatoren gewählt aus der Gruppe
- der Fettalkoholethoxylate der allgemeinen Formel R-O-(-CH₂-CH₂-O-)ₙ-H, wobei R einen verzweigten oder unverzweigten Alkyl-, Aryl- oder Alkenylrest und n eine Zahl von 10 bis 50 darstellen
- der ethoxylierten Wollwachsalkohole,
- der Polyethylenglycolether der allgemeinen Formel R-O-(-CH₂-CH₂-O-)ₙ-R', wobei R und R' unabhängig voneinander verzweigte oder unverzweigte Alkyl- oder Alkenylreste und n eine Zahl von 10 bis 80 darstellen
- der Fettsäureethoxylate der allgemeinen Formel
   R-COO-(-CH₂-CH₂-O-)ₙ -H, wobei R einen verzweigten oder unverzweigten Alkyl- oder Alkenylrest und n eine Zahl von 10 bis 40 darstellen,
- der veretherten Fettsäureethoxylate der allgemeinen Formel
   R-COO-(-CH₂-CH₂-O-)ₙ -R', wobei R und R' unabhängig voneinander verzweigte oder unverzweigte Alkyl- oder Alkenylreste und n eine Zahl von 10 bis 80 darstellen,
- der veresterten Fettsäureethoxylate der allgemeinen Formel
   R-COO-(-CH₂-CH₂-O-)ₙ -C(O)-R', wobei R und R' unabhängig voneinander verzweigte oder unverzweigte Alkyl- oder Alkenylreste und n eine Zahl von 10 bis 80 darstellen,
- der Polyethylenglycolglycerinfettsäureester gesättigter und/oder ungesättigter, verzweigter und/oder unverzweigter Fettsäuren und einem Ethoxylierungsgrad zwischen 3 und 50,
- der ethoxylierten Sorbitanester mit einem Ethoxylierungsgrad von 3 bis 100
- der Cholesterinethoxylate mit einem Ethoxylierungsgrad zwischen 3 und 50,
- der ethoxylierten Triglyceride mit einem Ethoxylierungsgrad zwischen 3 und 150,
- der Alkylethercarbonsäuren der allgemeinen Formel
   R-O-(-CH₂-CH₂-O-)ₙ-CH₂-COOH bzw. deren kosmetisch oder pharmazeutisch akzeptablen Salze, wobei R einen verzweigten oder unverzweigten Alkyl- oder Alkenylrest mit 5-30 C-Atomen und n eine Zahl von 5 bis 30 darstellen,
- der Polyoxyethylensorbitolfettsäureester, basierend auf verzweigten oder unverzweigten Alkan- oder Alkensäuren und einen Ethoxylierungsgrad von 5 bis 100 aufweisend, beispielsweise vom Sorbeth-Typ,
- der Alkylethersulfate bzw. die diesen Sulfaten zugrundeliegenden Säuren der allgemeinen Formel R-O-(-CH₂-CH₂-O-)ₙ-SO₃-H mit kosmetisch oder pharmazeutisch akzeptablen Kationen, wobei R einen verzweigten oder unverzweigten Alkyl- oder Alkenylrest mit 5-30 C-Atomen und n eine Zahl von 1 bis 50 darstellen.
- der Fettalkoholpropoxylate der allgemeinen Formel
   R-O-(-CH₂-CH(CH₃)-O-)ₙ-H, wobei R einen verzweigten oder unverzweigten Alkyl- oder Alkenylrest und n eine Zahl von 10 bis 80 darstellen,
- der Polypropylenglycolether der allgemeinen Formel
   R-O-(-CH₂-CH(CH₃)-O-)ₙ-R', wobei R und R' unabhängig voneinander verzweigte oder unverzweigte Alkyl- oder Alkenylreste und n eine Zahl von 10 bis 80 darstellen
- der propoxylierten Wollwachsalkohole,
- der veretherten Fettsäurepropoxylate der allgemeinen Formel
   R-COO-(-CH₂-CH(CH₃)-O-)ₙ-R', wobei R und R' unabhängig voneinander verzweigte oder unverzweigte Alkyl- oder Alkenylreste und n eine Zahl von 10 bis 80 darstellen,
- der veresterten Fettsäurepropoxylate der allgemeinen Formel
   R-COO-(-CH₂-CH(CH₃)-O-)ₙC(O)-R', wobei R und R' unabhängig voneinander verzweigte oder unverzweigte Alkyl- oder Alkenylreste und n eine Zahl von 10 bis 80 darstellen,
- der Fettsäurepropoxylate der allgemeinen Formel
   R-COO-(-CH₂-CH(CH₃)-O-)ₙ-H, wobei R einen verzweigten oder unverzweigten Alkyl- oder Alkenylrest und n eine Zahl von 10 bis 80 darstellen,
- der Polypropylenglycolglycerinfettsäureester gesättigter und/oder ungesättigter, verzweigter und/oder unverzweigter Fettsäuren und einem Propoxylierungsgrad zwischen 3 und 80
- der propoxylierten Sorbitanester mit einem Propoxylierungsgrad von 3 bis 100
- der Cholesterinpropoxylate mit einem Propoxylierungsgrad von 3 bis 100
- der propoxylierten Triglyceride mit einem Propoxylierungsgrad von 3 bis 100
- der Alkylethercarbonsäuren der allgemeinen Formel
   R-O-(-CH₂-CH(CH₃)O-)ₙ-CH₂-COOH bzw. deren kosmetisch oder pharmazeutisch akzeptablen Salze, wobei R einen verzweigten oder unverzweigten Alkyl- oder Alkenylrest und n eine Zahl von 3 bis 50 darstellen,
- der Alkylethersulfate bzw. die diesen Sulfaten zugrundeliegenden Säuren der allgemeinen Formel R-O-(-CH₂-CH(CH₃)-O-)ₙ-SO₃-H mit kosmetisch oder pharmazeutisch akzeptablen Kationen, wobei R einen verzweigten oder unverzweigten Alkyl- oder Alkenylrest mit 5 - 30 C-Atomen und n eine Zahl von 1 bis 50 darstellen,
- der Fettalkoholethoxylate/propoxylate der allgemeinen Formel
   R-O-Xₙ-Yₘ-H, wobei R einen verzweigten oder unverzweigten Alkyl- oder Alkenylrest darstellen, wobei X und Y nicht identisch sind und jeweils entweder eine Oxyethylengruppe oder eine Oxypropylengruppe und n und m unabhängig voneinander Zahlen von 5 bis 50 darstellen,
- der Polypropylenglycolether der allgemeinen Formel
   R-O-Xₙ-Yₘ-R', wobei R und R' unabhängig voneinander verzweigte oder unverzweigte Alkyl- oder Alkenylreste darstellen, wobei X und Y nicht identisch sind und jeweils entweder eine Oxyethylengruppe oder eine Oxypropylengruppe und n und m unabhängig voneinander Zahlen von 5 bis 100 darstellen,
- der veretherten Fettsäurepropoxylate der allgemeinen Formel
   R-COO-Xₙ-Yₘ-R', wobei R und R' unabhängig voneinander verzweigte oder unverzweigte Alkyl- oder Alkenylreste darstellen, wobei X und Y nicht identisch sind und jeweils entweder eine Oxyethylengruppe oder eine Oxypropylengruppe und n und m unabhängig voneinander Zahlen von 5 bis 100 darstellen,
- der Fettsäureethoxylate/propoxylate der allgemeinen Formel
   R-COO-Xₙ-Yₘ=H, wobei R einen verzweigten oder unverzweigten Alkyl- oder Alkenylrest, wobei X und Y nicht identisch sind und jeweils entweder eine Oxyethylengruppe oder eine Oxypropylengruppe und n und m unabhängig voneinander Zahlen von 5 bis 50 darstellen.

Insbesondere ist vorteilhaft, wenn der polyethoxylierte bzw. polypropoxylierte bzw. polyethoxylierte und polypropoxylierte O/W-Emulgator oder die polyethoxylierten bzw. polypropoxylierten bzw. polyethoxylierten und polypropoxylierten O/W-Emulgatoren gewählt wird oder werden aus der Gruppe
- der Fettalkoholethoxylate der allgemeinen Formel R-O-(-CH₂-CH₂-O-)ₙ-H, wobei R einen verzweigten oder unverzweigten Alkyl- oder Alkenylrest mit 5 - 30 C-Atomen und n eine Zahl von 10 bis 25 darstellen
- der ethoxylierten Wollwachsalkohole mit HLB-Werten von 8 - 17, ganz besonders vorteilhaft mit HLB-Werten von 12- 16.
- der Polyethylenglycolether der allgemeinen Formel R-O-(-CH₂-CH₂-O-)ₙ-R', wobei R und R' unabhängig voneinander verzweigte oder unverzweigte Alkyl- oder Alkenylreste mit 5 - 30 C-Atomen und n eine Zahl von 10 bis 25 darstellen,
- der Fettsäureethoxylate der allgemeinen Formel R-COO-(-CH₂-CH₂-O-)ₙ -H, wobei R einen verzweigten oder unverzweigten Alkyl- oder Alkenylrest mit 5 - 30 C-Atomen und n eine Zahl von 10 bis 25 darstellen,
- der veretherten Fettsäureethoxylate der allgemeinen Formel
   R-COO-(-CH₂-CH₂-O-)ₙ-R', wobei R und R' unabhängig voneinander verzweigte oder unverzweigte Alkyl- oder Alkenylreste mit 5 - 30 C-Atomen und n eine Zahl von 10 bis 50 darstellen,
- der veresterten Fettsäureethoxylate der allgemeinen Formel
   R-COO-(-CH₂-CH₂-O-)ₙ -C(O)-R', wobei R und R' unabhängig voneinander verzweigte oder unverzweigte Alkyl- oder Alkenylreste mit 5 - 30 C-Atomen und n eine Zahl von 10 bis 50 darstellen,
- der Polyethylenglycolglycerinfettsäureester gesättigter und/oder ungesättigter, verzweigter und/oder unverzweigter Fettsäuren mit 6 bis 26 C-Atomen und einem Ethoxylierungsgrad zwischen 3 und 40
- der ethoxylierten Sorbitanester mit einem Ethoxylierungsgrad von 3 bis 30
- der Cholesterinethoxylate mit HLB-Werten von 8 - 17, ganz besonders vorteilhaft mit HLB-Werten von 12 - 16
- der ethoxylierten Triglyceride mit HLB-Werten von 8 - 17, ganz besonders vorteilhaft mit mit HLB-Werten von 12 - 16
- der Alkylethercarbonsäuren der allgemeinen Formel
   R-O-(-CH₂-CH₂-O-)ₙ-CH₂-COOH bzw. deren kosmetisch oder pharmazeutisch akzeptablen Salze, wobei R einen verzweigten oder unverzweigten Alkyl- oder Alkenylrest mit 5 - 30 C-Atomen und n eine Zahl von 10 bis 20 darstellen,
- der Polyoxyethylensorbitolfettsäureester, basierend auf verzweigten oder unverzweigten Alkan- oder Alkensäuren und einen Ethoxylierungsgrad von 10 bis 80 aufweisend, beispielsweise vom Sorbeth-Typ,
- der Alkylethersulfate bzw. die diesen Sulfaten zugrundeliegenden Säuren der allgemeinen Formel R-O-(-CH₂-CH₂-O-)ₙ -SO₃-H mit kosmetisch oder pharmazeutisch akzeptablen Kationen, wobei R einen verzweigten oder unverzweigten Alkyl- oder Alkenylrest mit 5 - 30 C-Atomen und n eine Zahl von 3 bis 30 darstellen,
- der Fettalkoholpropoxylate der allgemeinen Formel
   R-O-(-CH₂-CH(CH₃)-O-)ₙ-H, wobei R einen verzweigten oder unverzweigten Alkyl- oder Alkenylrest mit 5 - 30 C-Atomen und n eine Zahl von 10 bis 30 darstellen,
- der Polypropylenglycolether der allgemeinen Formel
   R-O-(-CH₂-CH(CH₃)-O-)ₙ-R', wobei R und R' unabhängig voneinander verzweigte oder unverzweigte Alkyl- oder Alkenylreste mit 5 - 30 C-Atomen und n eine Zahl von 10 bis 40 darstellen,
- der propoxylierten Wollwachsalkohole mit HLB-Werten von 8 - 17 ganz besonders vorteilhaft mit HLB-Werten von 12-16,
- der Fettsäurepropoxylate der allgemeinen Formel
   R-COO-(-CH₂-CH(CH₃)-O-)ₙ-H, wobei R einen verzweigten oder unverzweigten Alkyl- oder Alkenylrest mit 5 - 30 C-Atomen und n eine Zahl von 10 bis 40 darstellen,
- der veretherten Fettsäurepropoxylate der allgemeinen Formel
   R-COO-(-CH₂-CH(CH₃)-O-)ₙ-R', wobei R und R' unabhängig voneinander verzweigte oder unverzweigte Alkyl- oder Alkenylreste mit 5 - 30 C-Atomen und n eine Zahl von 10 bis 30 darstellen,
- der veresterten Fettsäurepropoxylate der allgemeinen Formel
   R-COO-(-CH₂-CH(CH₃)-O-)ₙ-C(O)-R', wobei R und R' unabhängig voneinander verzweigte oder unverzweigte Alkyl- oder Alkenylreste mit 5 - 30 C-Atomen und n eine Zahl von 10 bis 50 darstellen,
- der Polypropylenglycolglycerinfettsäureester gesättigter und/oder ungesättigter, verzweigter und/oder unverzweigter Fettsäuren mit 6 bis 26 C-Atomen und einem Propoxylierungsgrad zwischen 3 und 50
- der propoxylierten Sorbitanester mit einem Propoxylierungsgrad von 3 bis 80
- der Cholesterinpropoxylate mit HLB-Werten von 8 - 17, ganz besonders vorteilhaft mit mit HLB-Werten von 12 16
- der propoxylierten Triglyceride mit HLB-Werten von 8 - 17 ganz besonders vorteilhaft mit mit HLB-Werten von 12 - 16
- der Alkylethercarbonsäuren der allgemeinen Formel
   R-O-(-CH₂-CH(CH₃)O-)ₙ-CH₂-COOH bzw. deren kosmetisch oder pharmazeutisch akzeptablen Salze, wobei R einen verzweigten oder unverzweigten Alkyl- oder Alkenylrest mit 5 - 30 C-Atomen und n eine Zahl von 10 bis 30 darstellen,
- der Alkylethersulfate bzw. die diesen Sulfaten zugrundeliegenden Säuren der allgemeinen Formel R-O-(-CH₂-CH(CH₃)-O-)ₙ-SO₃-H mit kosmetisch oder pharmazeutisch akzeptablen Kationen, wobei R einen verzweigten oder unverzweigten Alkyl- oder Alkenylrest mit 5 - 30 C-Atomen und n eine Zahl von 1 bis 30 darstellen.

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 8-17 ganz besonders vorteilhaft mit mit HLB-Werten von 12 - 16, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Octydodecylalkohole, Stearylalkohole, Cetylalkohole, Cetylstearylalkohole (Cetearylalkohole), Behenylalkohle zu wählen. Insbesondere bevorzugt sind:
Polyethylenglycol(16)octyldodecylether (Octyldodeceth-16), Polyethylenglycol(20)octyldodecylether (Octyldodeceth-20), Polyethylenglycol(25)octyl-dodecylether (Octyldodeceth-25), Polyethylenglycol(13)stearylether (Steareth-13), Polyethylenglycol(14)stearylether (Steareth-14), Polyethylenglycol(15)stearylether (Steareth-15), Polyethylenglycol(16)-stearylether (Steareth-16), Polyethylenglycol(17)stearylether (Steareth-1 7), Polyethylenglycol(18)stearylether (Steareth-18), Polyethylenglycol(19)stearylether (Steareth-19), Polyethylenglycol(20)stearylether (Steareth-20),
Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(1 5)isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-1 8), Polyethylenglycol(19)isostearylether (Isosteareth-19-), Polyethylenglycol(20)isostearylether (Isosteareth-20),
Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),
Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)-isocetylether (Isoceteth-16), Polyethylenglycol(17)isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20),
Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15),
Polyethylenglycol(12)laurylether (Laureth-12), Polyethylenglycol(12)isolaurylether (Isolaureth-12),
Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(1 7)cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20), Polyethylenglycol(10)behenylether (Beheneth-10), Polyethylenglycol(20)behenylether (Beheneth-20), Polyethylenglycol(25)behenylether (Beheneth-25), Polyethylenglycol(30)behenylether (Beheneth-30).

Es ist ferner von Vorteil, die Fettsäureethoxylate aus folgender Gruppe zu wählen:
Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat,
Polyethylenglycol(12)isostearat, Polyethylenglycol(13)isostearat, Polyethylenglycol(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol(22)isostearat, Polyethylenglycol(23)isostearat, Polyethylenglycol(24)isostearat, Polyethylenglycol(25)isostearat,
Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat.
Als ethoxylierte Alkylethercarbonsäure bzw. deren Salz kann vorteilhaft das Natriumlaureth-11-carboxylat verwendet werden.

Als Alkylethersulfat kann Natriumlaureth-14-sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als e thoxylierte T riglyceride k önnen v orteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)-sorbitanmonooleat zu wählen.

Als fakultative, dennoch erfindungsgemäß vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkohole einer Kettenlänge von 8 bis 24, insbesondere 12 - 18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Es ist erfindungsgemäß möglich, den Gesamtgehalt an Emulgatoren kleiner als 20 Gew.-%; bezogen auf das Gesamtgewicht des Mikroemulsionsgels, zu halten. Es wird bevorzugt, den Gesamtgehalt an Emulgatoren zwischen 10 Gew.-% und 14 Gew.-%, bezogen auf das Gesamtgewicht des Emulsionsgels, zu halten.

Die Ölphase der erfindungsgemäßen Emulsionsgele wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Dicaprylylcarbonat, Octyldodecanol, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher ÖI- und Wachskomponenten sind vorteilhaftim Sinne der vorliegenden Erfindung einzusetzen.

Es kann auch gegebenenfalls vorteilhaft sein, Wachse, beispielsweise Cetylpalmitat, als alleinige Lipidkomponente der Ölphase einzusetzen. In solchen Fällen können die erfindungsgemäßen O/W-Mikroemulsionsgele auch gegebenenfalls als Mikrodispersionen fester Wachspartikel anfallen.

Vorteilhaft wird die Ölphase gewählt aus der Gruppe Ethylhexylpalmitat, 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether, Dicaprylylcarbonat.

Besonders vorteilhaft sind Mischungen aus Ethylhexylpalmitat und Dicaprylylcarbonat, Ethylhexylpalmitat und 2-Ethylhexylisostearat, Mischungen aus Ethylhexylpalmitat und Isotridecylisononanoat sowie Mischungen aus Ethylhexylpalmitat, Octyldodecanol, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Decamethylcyclopentasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan,Octamethylcyclotetrasiloxan, Polydimethylsiloxan, Poly(methylphenylsiloxan).

Besonders vorteilhaft sind ferner Mischungen aus Cyclomethicon und Isotridecylisononanoat, aus Cyclomethicone und Ethylhexylpalmitat, Cyclomethicon und 2-Ethylhexylisostearat, Cyclomethicon und Octyldodecanol.

Die Polyole, insbesondere die Triole, die in der Erfindung als Vernetzersubstanzen wirken, bilden ein eingenständiges Gelnetzwerk und halten das Netzwerk und die Emulsionströpfchen in den Knotenpunkten des Netzes zusammen. Die Polyole tragen auch die optische Transparenz der Emulsionsgele bei.
Vorteilhaft wird das Polyol gewählt aus der Gruppen kurzkettiger, verzweigten und/oder unverzweigten Diole, Sorbitole und Triole. Vorteilhaft sind Glycerin, Propylenglykol, Dipropylenglykol, Sorbitol, Butylenglykol.
Besonders vorteilhaft ist Glycerin, Mischungen aus Glycerin und Propylenglykol, Mischung aus Glycerin und Dipropylenglykol, Mischung aus Glycerin und Sorbitol, Mischung aus Glycerin Butylenglykol.

Die Herstellung der erfindungsgemäßen transparenten Gele erfolgt vorteilhaft dergestalt, dass man ein Gemisch aus den Grundkomponenten, umfassend Wasserphase, Ölphase, einen oder mehrere der erfindungsgemäßen O/W-Emulgatoren, gewünschtenfalls einen oder mehrere W/O-Emulgatoren, sowie gewünschtenfalls weitere Hilfs-, Zusatz- und/oder Wirkstoffe, welche auf eine Temperatur bringt, bis alle Komponente aufgelöst sind. Das gebildete Emulsionsgel hernach wird auf Raumtemperatur abkühlt. Dies geschieht bevorzugt unter Rühren.

Erstaunlicherweise ist es jeweils möglich, auf einen Homogenisierungsschritt zu verzichten.

Vorteilhaft lassen sich große Mengen saurer Aluminium- und/oder Aluminium/Zirkoniumsalze stabil in die Emulsionen einarbeiten. Es können 5 bis 40 Gew.-%, insbesondere 7 bis 25 Gew.-% Aluminiumchlorhydrat und/oder Aluminium/Zirkoniumchlorhydrat stabil in die Emulsionen eingearbeitet werden. Hierbei beziehen sich die beschriebenen Konzentrationsbereiche auf die sog. Aktivgehalte der Antitranspirant-Komplexe: bei den A luminium-Verbindungen auf wasserfreie Komplexe, bei d en Aluminium/Zirkonium-Verbindungen auf wasser- und pufferfreie Komplexe. Als Puffer wird hier üblicherweise Glycin verwendet.

Die nachfolgende Auflistung vorteilhaft einzusetzender Antitranspirant-Wirker soll in keinster Weise einschränkend sein:
Aluminium-Salze (der empirischen Summenformel [Al₂(OH)ₘClₙ], wobei m+n=6):
   - Aluminium-Salze wie Aluminiumchlorid AlCl₃, Aluminiumsulfat Al₂(SO₄)₃
   - Aluminiumchlorhydrat [Al₂(OH)₅Cl] x H₂O
      Standard Al-Komplexe: Locron L (Clariant), Chlorhydrol (Reheis), ACH-303 (Summit), Aloxicoll L (Giulini).
      Aktivierte Al-Komplexe: Reach 501 (Reheis), AACH-324 (Summit)
   - Aluminiumsesquichlorhydrat [Al₂(OH)_{4,5}Cl_{1,5}] x H₂O
      Standard AI-Komplexe: Aluminum Sesquichlorohydrate (Reheis), ACH-308 (Summit), Aloxicoll 31L (Giulini)
      Aktivierte Al-Komplexe: Reach 301 (Reheis)
   - Aluminiumdichlorhydrat [Al₂(OH)₄Cl₂] x H₂O
Aluminium-Zirkonium-Salze:
   - Aluminium/Zirkonium Trichlorhydrex Glycin [Al₄Zr(OH)₁₃Cl₃] x H₂O x Gly Standard Al/Zr-Komplexe: Rezal 33GP (Reheis), AZG-7164 (Summit), Zirkonal P3G (Giulini)
      Aktivierte Al/Zr-Komplexe: Reach AZZ 902 (Reheis), AAZG-7160 (Summit), Zirkonal AP3G (Giulini)
   - Aluminium/Zirkonium Tetrachlorhydrex Glycin [Al₄Zr(OH)₁₂Cl₄] x H₂O x Gly Standard AI/Zr-Komplexe: Rezal 36G (Reheis), AZG-368 (Summit), Zirkonal L435G (Giulini)
      Aktivierte AI/Zr-Komplexe: Reach AZP 855 (Reheis), AAZG-6313-15 (Summit), Zirkonal AP4G (Giulini)
   - Aluminium/Zirkonium Pentachlorhydrex Glycin [Al₈Zr(OH)₂₃Cl₅] x H₂O x Gly Standard Al/Zr-Komplexe: Rezal 67 (Reheis), Zirkonal L540 (Giulini)
      Aktivierte AI/Zr-Komplexe: Reach AZN 885 (Reheis)
   - Aluminium/Zirkonium Octachlorhydrex Glycin [Al₈Zr(OH)₂₀Cl₈] x H₂O x Gly

Ebenso von Vorteil können aber auch Glycin-freie Aluminium/Zirkonium-Salze sein.

Dabei soll die Verwendung der Antitranspirant-Wirker aus den Rohstoffklassen Aluminium- und Aluminium/Zirkonium-Salzen nicht auf die handelsüblichen zumeist wäßrigen Lösungen, wie z.B. Locron L (Clariant), beschränkt sein, sondern es kann auch von Vorteil sein, die ebenfalls handelsüblichen wasserfreien Pulver derselbigen Rohstoffe durch Einbringung in die beanspruchten Formulierungen zum Einsatz zu bringen, wie z.B. Locron P (Clariant).

Vorteilhaft könnte auch die Verwendung von sog. AT-Salz Suspensionen sein, bei denen pulverförmig vorliegende Aluminium- und Aluminium/Zirkonium-Salze in diversen Ölen dispergiert angeboten werden.

Desweiteren kann es aber auch von Vorteil sein, spezielle Aluminium- und Aluminium/Zirkonium-Salze zum Einsatz zu bringen, die zur Löslichkeitsverbesserung als Glykol-Komplexe angeboten werden.

Weitere vorteilhafte Antitranspirant-Wirker basieren anstelle von Aluminium bzw. Zirkonium auf anderen Metallen, wie z.B. Beryllium, Titan, Hafnium.

Dabei soll die Liste der verwendbaren Antitranspirant-Wirker aber nicht auf metallhaltige Rohstoffe begrenzt sein, sondern von Vorteil sind auch Verbindungen, die Nichtmetalle wie Bor enthalten sowie solche, die dem Bereich der organischen Chemie zuzurechnen sind, wie z.B. Anticholinergika.
Vorteilhaft sind in diesem Sinne auch Polymere, die sowohl metallhaltig als auch metallfrei sein können.

Der in zahlreichen Zubereitungen auftretende Effekt, dass nach dem Auftragen der Zubereitung auf der Haut ein sichtbarer weißlicher Rückstand zurückbleibt, wird in der Regel vom Anwender als störend empfunden. In wasserfreien Zubereitungen hat sich der Einsatz von propoxylierten Alkoholen zur Kaschierung dieser Erscheinung bewährt. Im Falle von wasserhaltigen Zubereitungen ist bisher keine zufriedenstellende Lösung dieses Problems bekannt. Der Zusatz von propoxylierten Alkoholen mit 10 bis 20 Propyloxyeinheiten und 2 bis 10 Kohlenstoffatomen in der Alkylkette, insbesondere PPG-14-Butylether, als Bestandteil der mittelpolaren Ölphase hilft dem beschriebenen Mangel des Standes der Technik ab, indem das Auftreten derartiger weißlicher Rückstände zuverlässig kaschiert wird.

Vorteilhaft können erfindungsgemäßen Zubereitungen Desodorantien zugesetzt werden. Den üblichen kosmetischen Desodorantien liegen unterschiedliche Wirkprinzipien zugrunde.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluß selbst wird dadurch nicht beeinflußt, im Idealfalle wird nur die mikrobielle Zersetzung des Schweißes zeitweilig gestoppt. Auch die Kombination von Adstringentien mit antimikrobiell wirksamen Stoffen in ein und derselben Zusammensetzung ist gebräuchlich.

Alle für Desodorantien gängigen Wirkstoffe können vorteilhaft genutzt werden, beispielsweise Geruchsüberdecker wie die gängigen Parfümbestandteile, Geruchsabsorber, beispielsweise die in der DE 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen Emulsionen eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatriën-1-ol) sowie die in den DE 37 40 186, DE 39 38 140, DE 42 04 321, DE 42 29 707, DE 42 29 737, DE 42 37 081, DE 43 09 372, DE 43 24 219 beschriebenen wirksamen Agenzien. Auch Natriumhydrogencarbonat ist vorteilhaft zu verwenden.

Die Liste der genannten Wirkstoffe bzw. Wirkstoffkombinationen, die in den erfindungsgemäßen Emulsionen verwendet werden können, soll selbstverständlich nicht limitierend sein.
Die Menge der Desodorantien (eine oder mehrere Verbindungen) in den Zubereitungen beträgt vorzugsweise 0,01 bis 10 Gew.-%, besonders bevorzugt 0,05 bis 5 Gew.-%, insbesondere 0,1 bis 1 Gew.-% bezogen auf das Gesamtgewicht der Zubereitung.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse, Antioxidantien, Vitamine und/oder dessen Derivate, Lichtschutzfilter oder andere übliche Bestandteile einer kosmetischen oder dermatologischen Formulierung wie Alkohole, Polyole, Polymere, Schaumstabilisatoren, organische Lösungsmittel oder Silikonderivate sowie Moisturizer.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht, und die Gesamtmenge bzw. auf das Gesamtgewicht der Zubereitungen bezogen.

### Beispielrezepturen für transparente Antitranspirantgele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| INCl-Nafme(n) | Bsp.1 | Bsp.2 | Bsp.3 | Bsp.4 | Bsp.5 |
|---|---|---|---|---|---|
| Octyldodeceth-20 | | | | | |
| Octyldodeceth-25 | 13,00 | 13,00 | 12,00 | 13,00 | 13,00 |
| Ceteareth-15 | | | | | |
| Cyclomethicone | | 40,00 | 15,00 | | |
| Dicaprylyl Carbonate | | | | | |
| Ethylhexyl Palmitate | 40,00 | | | 40,00 | 40,00 |
| Octyldodecanol | | | 15,00 | | |
| Glycerin | 15,00 | 15,00 | 15,00 | 13,00 | 13,00 |
| Butylene Glycol | | | | | |
| Aluminum Chlorohydrate (50% Lösung) | 20,00 | 20,00 | 20,00 | 20,00 | 15,00 |
| Water | 12,00 | 12,00 | 23,00 | 14,00 | 19,00 |
| Summen: | 100,00 | 100,00 | 100,00 | 100,00 | 100,00 |
| | | | | | |

| INCl-Name(n) | Bsp.6 | Bsp.7 | Bsp.8 | Bsp.9 | Bsp.10 |
|---|---|---|---|---|---|
| Octyldodeceth-20 | | | 13,00 | | |
| Octyldodeceth-25 | 13,00 | 12,00 | | | |
| Ceteareth-15 | | | | 12,00 | 13,00 |
| Cyclomethicone | | | | 15,00 | |
| Dicaprylyl Carbonate | | 35,00 | | | |
| Ethylhexyl Palmitate | 40,00 | | 40,00 | | 40,00 |
| Octyldodecanol | | | | 15,00 | |
| Glycerin | | | 15,00 | 15,00 | 15,00 |
| Butylene Glycol | 13,00 | 15,00 | | | |
| Aluminum Chlorohydrate (50% Lösung) | 20,00 | 20,00 | 20,00 | 30,00 | 20,00 |
| Water | 14,00 | 18,00 | 12,00 | 13,00 | 12,00 |
| Summen: | 100,00 | 100,00 | 100,00 | 100,00 | 100,00 |

## Patentansprüche

1. Kosmetische und/oder dermatologische Zubereitung zur Verhinderung der übermäßigen Geruchs- und Schweißabsonderung der Haut, bestehend aus einem optisch transluzenten bis transparenten, alkoholfreien Emulsionsgel, insbesondere Mikroemulsionsgel vom Typ ÖI-in-Wasser (O/W), umfassend eine Ölphase und eine Wasserphase, enthaltend:
- einen oder mehrere polyethoxylierte O/W-Emulgatoren und/oder einen oder mehrere polypropoxylierte O/W-Emulgatoren und/oder ein oder mehrere Polyole
- gewünschtenfalls ferner enthaltend einen oder mehrere W/O-Emulgatoren
- einen Emulgatorgehalt kleiner als 20 Gew.-%, bezogen auf das Gesamtgewicht der Emulsion,
- einen Antitranspirantgehalt von 5 bis 40 Gew-%, bezogen auf das Gesamtgewicht der Emulsion,
- einen Polyolgehalt von 1 bis 30 Gew.-%, bezogen auf das Gesamtgewicht der Emulsion,
erhältlich auf die Weise, dass ein Gemisch aus den Grundkomponenten, umfassend Wasserphase, Ölphase, einen oder mehrere der erfindungsgemäßen O/W-Emulgatoren, gewünschtenfalls einen oder mehrere W/O-Emulgatoren, sowie gewünschtenfalls weitere Hilfs-, Zusatz- und/oder Wirkstoffe aufwärmt bis alle Komponente aufgelöst sind und hernach auf Raumtemperatur unter Rühren abkühlt, ohne die Emulsion zu homogenisieren
und bei welcher die Tröpfchen der diskontinuierlichen Ölphase durch Polyole miteinander verbunden sind.

2. Kosmetische und/oder dermatologische Zubereitung nach Anspruch 1, **dadurch gekennzeichnet, dass** die ÖI-in-Wasser-Emulsion optisch klar transluzent bis transparent ist und insbesondere eine mittlere Tröpfchengröße kleiner 100 nm aufweist.

3. Kosmetische und/oder dermatologische Zubereitung nach einem der vorhergehenden Ansprüche, **dadurch gekennzeichnet, dass** die Viskosität zwischen 5,000 bis 500,000 mPa s, vorteilhaft zwischen 5,000 bis 400,000 mPa s, besonders vorteilhaft zwischen 10,000 bis 300,000 mPa s, liegt

4. Kosmetische und/oder dermatologische Zubereitung nach Anspruch 1, **dadurch gekennzeichnet, dass** die Antitranspirant-Wirkstoffe saure Salze sind.

5. Kosmetische und/oder dermatologische Zubereitung nach einem oder mehreren der vorhergehenden Ansprüche, **dadurch gekennzeichnet, dass** die Antitranspirantwirkstoffe eine oder mehrere saure Aluminium- und/oder Aluminium/Zirkoniumsalze sind und die Gesamtmenge an dem oder den sauren Aluminium- und/oder Aluminium/Zirkoniumsalzen mindestens 5 Gew.-% bezogen auf das Gesamtgewicht der Zubereitungen beträgt.

6. Kosmetische und/oder dermatologische Zubereitung nach mindestens einem der vorangehenden Ansprüche, **dadurch gekennzeichnet, dass** der Antitranspirantgehalt 5 bis 40 Gew.-%, insbesondere 10 bis 30 Gew.-%, bezogen auf das das Gesamtgewicht der Zubereitungen, beträgt.

7. Kosmetische und/oder dermatologische Zubereitung nach einem oder mehreren der vorhergehenden Ansprüche, **dadurch gekennzeichnet, dass** die Gesamtmenge an einem oder mehreren polyethoxylierten O/W-Emulgatoren in den fertigen Zubereitungen aus dem Bereich von 0,1 bis 20 Gew.-%, bevorzugt von 0,5 bis 18 Gew.-%, besonders bevorzugt von 1 bis 14 Gew.-% gewählt wird, bezogen auf das Gesamtgewicht der Zubereitungen.

8. Kosmetische und/oder dermatologische Zubereitung nach einem oder mehreren der vorhergehenden Ansprüche, **dadurch gekennzeichnet, dass** die Gesamtmenge an einem oder mehreren polypropoxylierten O/W-Emulgatoren in den fertigen Zubereitungen aus dem Bereich von 0,1 bis 20 Gew.-%, bevorzugt von 0,5 bis 18 Gew.-%, besonders bevorzugt von 1 bis 14 Gew.-% gewählt wird, bezogen auf das Gesamtgewicht der Zubereitungen.

9. Kosmetische und/oder dermatologische Zubereitung nach einem oder mehreren der vorhergehenden Ansprüche, **dadurch gekennzeichnet, dass** die Gesamtmenge an einem oder mehreren polyethoxylierten und polypropoxylierten O/W-Emulgatoren in den fertigen Zubereitungen aus dem Bereich von 0,1 bis 20 Gew.-%, bevorzugt von 0,5 bis 18 Gew.-%, besonders bevorzugt von 1 bis 14 Gew.-% gewählt wird, bezogen auf das Gesamtgewicht der Zubereitungen.

10. Kosmetische und/oder dermatologische Zubereitung nach einem oder mehreren der vorhergehenden Ansprüche, **dadurch gekennzeichnet, dass** die Gesamtmenge an einem oder mehreren W/O-Emulgatoren in den fertigen Zubereitungen aus dem Bereich von 0,1 bis 5 Gew.-%, bevorzugt von 0,5 bis 3,5 Gew.-%, besonders bevorzugt von 1 bis 2,5 Gew.-% gewählt wird, bezogen auf das Gesamtgewicht der Zubereitungen.

11. Kosmetische und/oder dermatologische Zubereitung nach einem oder mehreren der vorhergehenden Ansprüche, **dadurch gekennzeichnet, dass** die Gesamtmenge an einem oder mehreren Vernetzern, die Polyole, in den fertigen Zubereitungen aus dem Bereich von 1 bis 30 Gew.-%, bevorzugt von 5 bis 25 Gew.-%, besonders bevorzugt von 8 bis 20 Gew.-% gewählt wird, bezogen auf das Gesamtgewicht der Zubereitungen.

12. Kosmetische und/oder dermatologische Zubereitung nach einem oder mehreren der vorhergehenden Ansprüche, **dadurch gekennzeichnet, dass** die Zubereitungen dickflüssig bis halbfest und bis hin zu cremefest sind und in einem für kosmetische Stifte geeigneten oder Deoroller-Behältnis oder einem Zerstäuberpumpen-Behältnis an den Verbraucher weitergegeben werden.

13. Kosmetische und/oder dermatologische Zubereitung nach einem oder mehreren der vorhergehenden Ansprüche, **dadurch gekennzeichnet, dass** neben anderen kosmetischen Zusatzstoffen Parfüm und/oder Duftstoffe enthält.

14. Verwendung einer Antitranspirantformulierung nach mindestens einem der vorhergehenden Ansprüche zur Auftragung auf die menschliche Haut, insbesondere zur Verminderung der Schweißbildung.
